# FASCICULE DE BREVET EUROPEEN

(11) **EP 1 339 501 B1**
(45) Date de publication et mention de la délivrance du brevet: **17.02.2010**
(21) Numéro de dépôt: 01986949.4
(22) Date de dépôt: 04.12.2001
(51) Int. Cl.: B05B 11/06, A61M 15/00

(54) **DISPOSITIF DE DISTRIBUTION DE PRODUIT FLUIDE OU PULVERULENT**
VORRICHTUNG ZUR ABGABE VON FLIESSFÄHIGEN MEDIEN
FLUID OR POWDERY PRODUCT DISPENSING DEVICE

(43) Date de publication de la demande: 03.09.2003
(73) Titulaire: Valois SAS, 27110 Le Neubourg (FR)
(72) Inventeur: STRADELLA, Giuseppe, I-16032 Camogli (IT)
(74) Mandataire: CAPRI
(86) Numéro de dépôt international: PCT/EP2001/015408
(87) Numéro de publication internationale: WO 2002/045866

(56) Documents cités:
- WO-A-92/06727
- DE-A- 19 502 725
- US-A- 4 017 007
- US-A- 5 568 884

## Description

La présente invention concerne un dispositif de distribution de produit fluide ou pulvérulent, et plus particulièrement un dispositif pour distribuer unitairement une dose de produit contenu dans un réservoir à l'aide d'un écoulement d'air sous pressions.

Un tel dispositif ayant les caractéristiques techniques du préambule de la revendication 1 est connu du Document DE 19 502 725.

Le document WO 99/46055 divulgue un dispositif dans lequel un élément de fermeture sphérique, qui obture la sortie du réservoir, est expulsé par l'écoulement d'air créé par une chasse d'air. Pour l'utilisation d'un dispositif de distribution de poudre plus particulièrement, la pression d'air nécessaire pour actionner le dispositif devra être suffisamment élevée pour garantir la distribution complète de la dose, ainsi que son fractionnement si cela est nécessaire. Dans le dispositif susmentionné, la pression d'air nécessaire pour actionner le dispositif est déterminée par la résistance donnée par la bille pour être expulsée. Cette résistance est relativement difficile à contrôler et à prédéterminer puisqu'elle est dépendante du frottement entre la bille et son siège cylindrique dans lequel elle est emmanchée pour obturer de manière étanche ledit réservoir. Il peut par conséquent être nécessaire de minimiser l'interférence entre la sphère et son siège cylindrique, ce qui peut évidemment altérer l'efficacité de l'obturation. De plus, il peut être nécessaire de minimiser la profondeur et le positionnement de la sphère dans son siège afin de faciliter son expulsion. II peut également être nécessaire de fournir une pression d'air relativement élevée qui n'est pas toujours facile à réaliser à l'aide d'un système de pompe ou d'un système de soufflet, notamment lorsque ces chasses d'air sont actionnées manuellement par le patient. De plus, la distribution, c'est à dire l'expulsion de la bille de son siège, peut se produire à des longueurs différentes de la course de la pompe ou du soufflet de la chasse d'air, de sorte que l'instant précis de la distribution du produit ne peut pas être toujours prédéterminé de manière exacte. Enfin, il y a une limitation dans le choix des matériaux pour la sphère et pour son siège.

La présente invention a pour but de fournir un dispositif de distribution de produit fluide ou pulvérulent qui ne reproduit pas les inconvénients susmentionnés.

La présente invention a donc pour but de fournir un dispositif de distribution de produit fluide ou pulvérulent, dans lequel l'expulsion de l'élément de fermeture qui obture de manière étanche le réservoir est indépendante de la pression d'air fournie par la chasse d'air.

La présente invention a également pour but de fournir un dispositif de distribution de produit fluide ou pulvérulent du type susmentionné, qui soit simple et peu coûteux à fabriquer et à assembler.

La présente invention a aussi pour but de fournir un tel dispositif de distribution de produit fluide ou pulvérulent qui permette l'utilisation d'une chasse d'air très simple et peu coûteuse, et ne nécessitant pas la création d'un écoulement d'air particulièrement puissant.

La présente invention a encore pour but de fournir un tel dispositif de distribution de produit fluide ou pulvérulent qui garantisse la distribution totale de la dose de produit, ainsi que son fractionnement, si cela est requis.

La présente a donc pour objet un dispositif de distribution de produit fluide ou pulvérulent tel que décrit dans la revendication 1. Des modes de réalisation avantageux sont décrits dans les revendications dépendantes.

D'autres caractéristiques et avantages de la présente invention apparaîtront plus clairement au cours de la description détaillée suivante de plusieurs modes de réalisation de celle-ci, en référence aux dessins joints, donnés à titre d'exemples non limitatifs, et sur lesquels :
- la figure 1 est une vue schématique en section transversale d'un dispositif de distribution de produit fluide ou pulvérulent, en position de repos ;
- la figure 2 est une vue similaire à celle de la figure 1, en position d'actionnement ;
- la figure 3 est une vue schématique en section transversale montrant une variante des figures 1 et 2, en position de repos ;
- la figure 4 est une similaire à celle de la figure 3, en position d'actionnement ;
- la figure 5 est une vue schématique en section transversale d'un dispositif de distribution de produit fluide ou pulvérulent selon un mode de réalisation de la présente invention, en position de repos ; et
- les figures 6, 7 et 8 sont des vues schématiques en section transversale d'un autre mode de réalisation de l'invention, respectivement en position de repos, en position après mélange et en position de distribution.

La présente invention, dans ses diverses variantes et modes de réalisation décrits ci-après, concerne plus particulièrement un dispositif du type de celui divulgué dans le document WO 99/46055.

Il est toutefois entendu que la présente invention ne se limite pas à ce type de dispositif, mais est au contraire applicable à tous les types de dispositifs de distribution de produit fluide et pulvérulents tels que définis dans la revendication 1.

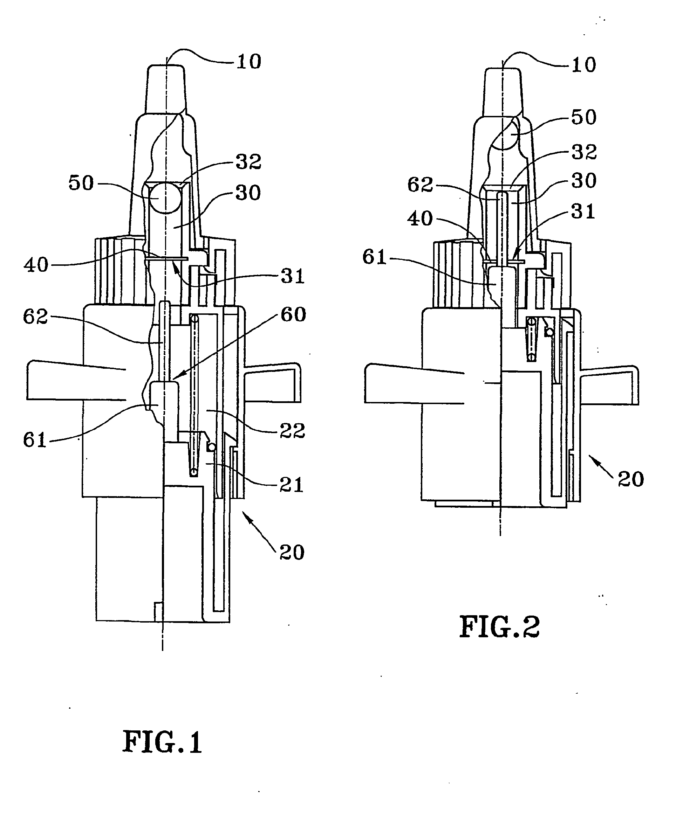

En référence aux figures 1 et 2, il est représenté un premier mode de réalisation. Le dispositif comporte un réservoir 30 comportant une entrée d'air 31 et une sortie de produit 32. L'entrée d'air 31 du réservoir est reliée à une chasse d'air 20 et la sortie de produit 32 du réservoir est reliée à une sortie de distribution 10 du dispositif. La sortie de produit 32 est obturée par un élément de fermeture sphérique 50, tel qu'une bille, qui est emmanchée à force dans ladite sortie de produit 32. L'entrée d'air 31 est pourvue d'un organe de retenue de produit 40 qui est adapté à maintenir le produit dans le réservoir avant l'actionnement du dispositif. La chasse d'air 20 est actionnée manuellement par l'utilisateur et est adaptée à créer un écoulement d'air qui va traverser le réservoir 30 pour emmener le produit qu'il contient en direction de la sortie de distribution 10.

Le dispositif comporte un système d'ouverture mécanique 60, qui est de préférence solidaire de la chasse d'air 20, c'est à dire qu'il est actionné simultanément à l'actionnement de ladite chasse d'air 20, et qui est adapté à coopérer avec ledit élément de fermeture 50 pour l'expulser mécaniquement de sa position d'obturation lors de l'actionnement du dispositif. Dans l'exemple représenté sur les figures 1 et 2, le système d'ouverture mécanique 60 comporte un ensemble de tige 61, 62, dont une première partie de tige 61 est solidaire de la chasse d'air 20, et une seconde partie de tige 62, de préférence de diamètre inférieur, est fixé à ladite première partie de tige 61 et est adapté à traverser, notamment à percer, l'organe de retenue de produit 40, puis à traverser le réservoir 30 jusqu'à coopérer avec l'élément de fermeture 50 pour l'expulser mécaniquement de sa position d'obturation.

L'organe de retenue de produit 40 peut être avantageusement réalisé sous la forme d'une membrane qui est étanche au produit mais perméable à l'air. De cette manière au début de l'actionnement de la chasse d'air 20, la pression augmente à la fois dans la chasse d'air et dans le réservoir 30, de sorte qu'au moment où la bille 50 est expulsée par la seconde partie de tige 62, le produit contenu dans le réservoir 30 est pulvérisé finement en direction de la sortie de distribution 10 du dispositif. Bien entendu, l'organe de retenue de produit 40 peut également être réalisé de manière étanche au produit et étanche à l'air, avant l'actionnement du dispositif, la pression d'air créée par la chasse d'air ne pénétrant à l'intérieur du réservoir 30 qu'au moment où ladite membrane 40 est percée.

Avantageusement, la première partie de tige 61 comporte au moins une rainure externe 63 de passage d'air adaptée à transmettre l'écoulement d'air au moment où la bille 50 est expulsée. Cette ou ces rainures peuvent être droites, c'est à dire axiales, ou hélicoïdales, afin de fournir le fractionnement de la poudre qui peut être éventuellement requis afin de réaliser une pulvérisation fine du produit, en particulier s'il s'agit d'une poudre.

La chasse d'air représentée sur les figures 1 et 2 comporte un piston 21 coulissant dans une chambre d'air 22, le piston 21 étant actionné manuellement par l'utilisateur. La présence de la tige, et en particulier de la première partie de tige 61 fournit un guidage pour ledit piston 21, ce qui facilite son actionnement, en imposant un déplacement axial dudit piston à l'intérieur de la chambre 22.

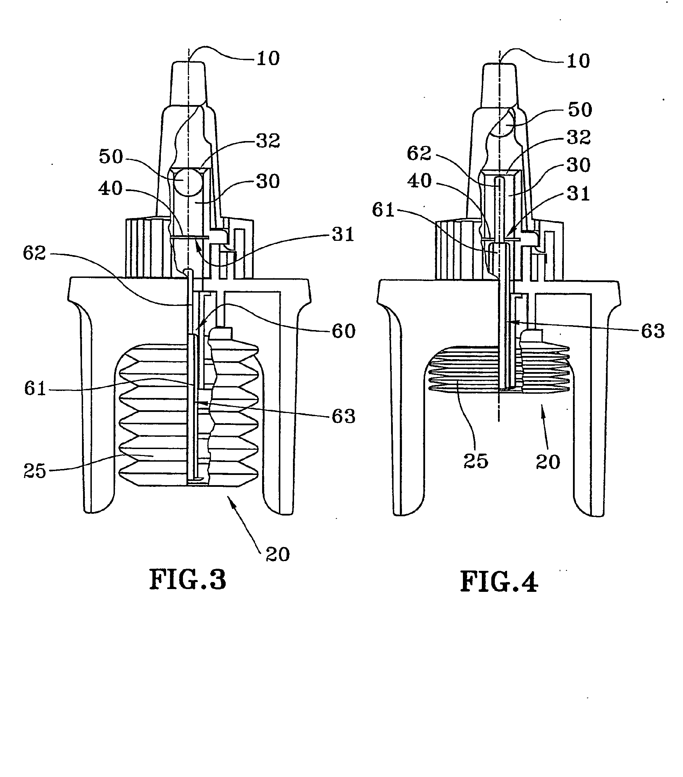

En référence aux figures 3 et 4, il est représenté une variante de réalisation du dispositif représenté sur les figures 1 et 2, dans laquelle la chasse d'air est réalisée sous la forme d'un soufflet 25. Ici également, la première partie de tige 61 fournit un guidage pour l'actionnement dudit soufflet 25, ce qui permet de réaliser ledit soufflet 25 de manière très simple, sans avoir à prévoir des moyens de guidage compliqués, puisque le guidage est déjà obtenu par ladite tige 61 disposée de manière centrale à l'intérieur dudit soufflet.

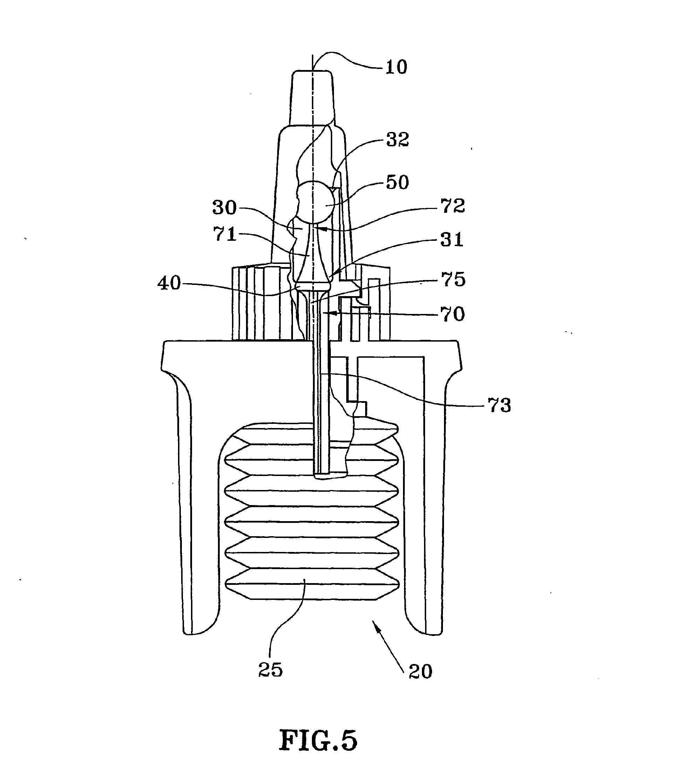

En référence à la figure 5, il est représenté un mode de réalisation de la présente invention. Dans ce mode de réalisation, et contrairement au mode de réalisation décrit précédemment, l'organe de retenue de produit 40 n'est pas destiné à être percé par le système d'ouverture mécanique 70, mais au contraire en forme une partie. Ainsi, ledit organe de retenue de produit 40 est avantageusement réalisé sous la forme d'un disque rigide qui se prolonge à l'intérieur du réservoir par une première tige 71 dont l'extrémité 72 coopère avec l'élément de fermeture 50. Cette première tige 71 est de préférence réalisée de manière conique, ou d'une forme environ similaire, qui permet d'une part de fournir suffisamment d'espace pour le produit contenu dans le réservoir, et d'autre part de garantir une bonne diffusion de l'écoulement d'air créé par la chasse d'air 20 au moment de l'expulsion du produit. De l'autre côté, l'organe de retenue de produit 40 se prolonge par une seconde tige 75 qui est solidaire de la chasse d'air 20. Cette seconde tige 75 est adaptée à déplacer l'organe de retenue 40, et par conséquent la première tige 71, et donc l'élément de fermeture 50 lors de l'actionnement de la chasse d'air 20. Avantageusement, le déplacement de la seconde tige 75 est réalisé en fin de course d'actionnement de la chasse d'air, afin de fournir un écoulement d'air sous pression au moment de l'expulsion de la bille 50.

Avantageusement, la seconde tige 75 comporte également une ou plusieurs rainures externes de passage d'air pour transmettre l'écoulement d'air à l'intérieur du réservoir 30 lorsque le dispositif est actionné. L'organe de retenue de produit 40 peut soit être complètement étanche au produit et à l'air dans sa position de repos, de sorte que l'écoulement d'air ne pénètre à l'intérieur du réservoir 30 qu'au moment où la bille 50 est expulsée, ou alors il peut comporter une ou plusieurs rainures de passage d'air, qui permettent à la pression de l'air de monter également à l'intérieur du réservoir 30 au début de l'actionnement de la chasse d'air 20, et jusqu'à l'expulsion de ladite bille 50. Une telle montée de pression dans le réservoir avant l'expulsion du produit peut également favoriser la fragmentation du produit, particulièrement lorsque le produit est une poudre.

Dans l'exemple de la figure 5, la chasse d'air 20 est un soufflet 25, avantageusement guidée par la seconde tige 75, comme expliqué précédemment, mais il est clair que tout type de chasse d'air est utilisable, et en particulier le système de piston représenté sur les figures 1 et 2.

La présente invention fournit donc un dispositif de distribution de produit fluide ou pulvérulent dans lequel il est possible d'augmenter l'interférence entre la bille et la sortie de produit 32 du réservoir 30, ce qui a pour conséquence une obturation plus sûre, en particulier en ce qui concerne l'étanchéité. D'autre part, du fait que l'interaction peut être plus importante et que l'expulsion de la bille n'est plus fournie par l'écoulement d'air, les matériaux les plus appropriés peuvent être choisis pour réaliser ladite bille et ledit siège prévus à la sortie de produit 32 du réservoir 30. D'autre part, on peut facilement ajuster la pression au moment de la distribution du produit, en modifiant simplement la longueur de la tige 61, 75 qui coopère avec la chasse d'air 20. Plus la tige est longue, plus tôt la bille 50 sera expulsée dans la course d'actionnement de la chasse d'air, et moins l'écoulement d'air sera important au moment de la distribution du produit.

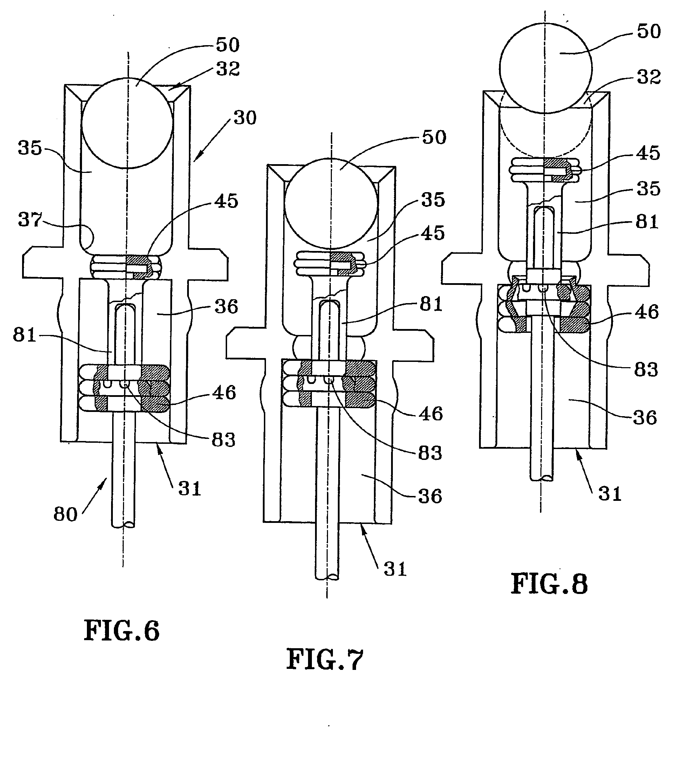

Bien que décrite principalement en liaison avec un produit pulvérulent, la présente invention s'applique également à la distribution de produits liquides. En particulier, comme représenté sur les figures 6 à 8, il est envisageable de l'appliquer à un dispositif comportant un réservoir 30 formé d'un réservoir de poudre 35 et d'un réservoir de liquide 36, ces deux produits étant destinés à être mélangés avant d'être distribués. Pour ce faire, le réservoir 30 est séparé au niveau d'une paroi de séparation (37) qui comporte une ouverture obturée au repos par un premier organe de retenue 45 qui sépare, de préférence de manière étanche, le réservoir de poudre 35 du réservoir de liquide 36. Un deuxième organe de retenue 46 est prévu dans le réservoir de liquide 36. Dans l'exemple représenté sur les figures, le réservoir supérieur est le réservoir de poudre 35 et le réservoir inférieur est le réservoir de liquide, mais ce pourrait être l'inverse. Le système d'ouverture mécanique 80 comporte une tige 81 qui est de préférence solidaire de la chasse d'air (non représentée). Cette tige entraîne d'abord les deux organes de retenue 45 et 46, de sorte que l'ouverture entre les deux réservoirs est libérée, et le liquide est transféré dans le réservoir de poudre 35 par l'action du second organe de retenue formant piston dans le réservoir de liquide 36. Dans la position de la figure 7, la totalité du liquide a été transférée dans le réservoir de poudre 35, dans lequel les deux matériaux se mélangent. Le second organe de retenue 46 ferme alors l'organe de retenue pour le mélange poudre/liquide. Le premier organe de retenue 45 est disposé très près de l'élément de fermeture 50. La tige 81 étant solidaire de la chasse d'air, le déplacement entre les figures 6 et 7 crée une compression d'air. Une continuation de la poussée de la tige 81 à partir de la position de la figure 7 a pour effet d'une part de déformer le second organe de retenue 46 de telle sorte que des ouvertures de passages d'air 83 prévues dans la tige 81 sont ouvertes pour relier la chasse d'air au réservoir 35. D'autre part, l'élément de fermeture 50 est expulsé mécaniquement par le premier organe de retenue 45, poussé par la tige 81. Ainsi, le mélange poudre/liquide est expulsé du réservoir 35, à travers la sortie de produit 32 du réservoir 30 au moyen de l'écoulement d'air comprimé créé par la chasse d'air.

L'invention peut également s'appliquer à un dispositif multidoses comportant plusieurs réservoirs individuels, des moyens étant alors prévus pour faire coopérer un réservoir respectivement avec la chasse d'air et le système d'ouverture mécanique, à chaque actionnement du dispositif.

La présente invention a été décrite en référence à plusieurs variantes, mais il est entendu qu'un homme du métier peut y apporter toutes modifications, sans sortir du cadre de la présente invention telle que définie par les revendications annexées.

## Revendications

1. Dispositif de distribution de produit fluide ou pulvérulent comportant une sortie de distribution (10), une chasse d'air (20) pour générer un écoulement d'air lors de l'actionnement du dispositif, et au moins un réservoir (30) contenant une dose unique de produit, ledit réservoir (30) comportant une entrée d'air (31) reliée à ladite chasse d'air (20) et une sortie de produit (32) reliée à ladite sortie de distribution (10), ladite entrée d'air (31) comportant un organe de retenue de produit (40) pour maintenir le produit dans le réservoir (30) jusqu'à la distribution du produit, et ladite sortie de produit (32) étant obturée par un élément de fermeture (50), ledit dispositif comportant un système d'ouverture mécanique (60, 70, 80) coopérant avec ledit élément de fermeture (50) pour l'expulser mécaniquement de sa position d'obturation lors de l'actionnement du dispositif, **caractérisé en ce que** l'élément de fermeture (50) est un élément sphérique, tel qu'une bille, emmanchée à force dans la sortie de produit (32) du réservoir (30), ledit système d'ouverture mécanique (70) étant solidaire dudit organe de retenue de produit (40).

2. Dispositif selon la revendication 1, dans lequel ledit système d'ouverture mécanique (60, 70, 80) est solidaire de ladite chasse d'air (20).

3. Dispositif selon la revendication 1 ou 2, dans lequel ledit organe de retenue de produit (40) est étanche au produit et étanche à l'air, avant l'actionnement du dispositif.

4. Dispositif selon la revendication 1 ou 2, dans lequel ledit organe de retenue de produit (40) est étanche au produit et perméable à l'air avant l'actionnement du dispositif.

5. Dispositif selon l'une quelconque des revendications précédentes, dans lequel ledit système d'ouverture mécanique (60) comporte une tige mobile (61, 62) adaptée à traverser ledit réservoir (30) lors de l'actionnement dudit dispositif pour expulser ledit élément de fermeture *(50).*

6. Dispositif selon l'une quelconque des revendications précédentes, dans lequel l'organe de retenue de produit (40) est une plaque qui se prolonge dans le réservoir (30) en direction de l'élément de fermeture (50) par une première tige (71), de préférence conique, dont l'extrémité (72) coopère avec l'élément de fermeture (50).

7. Dispositif selon la revendication 6, dans lequel ladite chasse d'air (20) comporte une seconde tige (75) qui coopère avec ledit organe de retenue (40) pour le déplacer ensemble avec la première tige (71) lors de l'actionnement du dispositif.

8. Dispositif selon la revendication 7, dans lequel ladite seconde tige (75) comporte au moins une rainure externe (73) de passage d'air.

9. Dispositif selon l'une quelconque des revendications précédentes, dans lequel l'organe de retenue de produit (40) comporte une ou plusieurs rainures de passage d'air.

10. Dispositif selon l'une quelconque des revendications précédentes, dans lequel le réservoir (30) comporte un réservoir de poudre (35) et un réservoir de liquide (36), et l'organe de retenue de produit (40) comporte un premier organe de retenue (45), qui au repos, sépare les deux réservoirs (35, 36), et un second organe de retenue (46), le système d'ouverture mécanique (80) comportant une tige (81) interconnectant lesdits deux organes de retenue (45, 46) de telle sorte que lors du déplacement de la tige (81), le liquide est d'abord transféré dans le réservoir de poudre (35) pour s'y mélanger avec la poudre, l'élément de fermeture (50) étant expulsé mécaniquement en fin de course de la tige (81) par ledit premier organe de retenue (45), des moyens de passage d'air (83) étant ouverts pour relier la chasse d'air au réservoir de poudre (35) pour réaliser l'expulsion du mélange poudre/liquide à travers la sortie de produit (32).

11. Dispositif selon l'une quelconque des revendications précédentes, dans lequel ledit système d'ouverture mécanique (60, 70, 80) forme un guidage pour l'actionnement manuel de la chasse d'air (20).

12. Dispositif selon l'une quelconque des revendications précédentes, dans lequel ladite chasse d'air (20) comporte un piston (21) coulissant dans une chambre d'air (22).

13. Dispositif selon l'une quelconque des revendications 1 à 11, dans lequel ladite chasse d'air comporte un soufflet (25).

## Claims

1. A device for dispensing a substance in fluid or powder form, the device comprising a dispensing outlet (10), an air blaster (20) for generating a blast of air when the device is actuated, and at least one reservoir (30) containing a single dose of substance, said reservoir (30) having an air inlet (31) connected to said air blaster (20), and a substance outlet (32) connected to said dispensing orifice (10), said air inlet (31) being provided with a substance retaining member (40) for retaining the substance in the reservoir (30) until it is dispensed, and said substance outlet (32) being closed off by a closure element (50), said device further comprising a mechanical opening system (60, 70, 80) co-operating with said closure element (50) for ejecting it mechanically from its closure position when the device is actuated, said dispenser device being **characterized in that** the closure element (50) is a spherical element, such as a ball, force fitted into the substance outlet (32) of the reservoir (30), said mechanical opening system (70) being secured to said substance retaining member (40).

2. A device according to claim 1, in which said mechanical opening system (60, 70, 80) is secured to said air blaster (20).

3. A device according to claim 1 or 2, in which said substance retaining member (40) is impermeable to the substance and is impermeable to air, before the device is actuated.

4. A device according to claim 1 or 2, in which said substance retaining member (40) is impermeable to the substance and permeable to air before the device is actuated.

5. A device according to any preceding claim, in which said mechanical opening system (60) comprises a movably mounted rod (61, 62) adapted to pass through said reservoir (30) when said device is actuated so as to eject said closure element (50).

6. A device according to any preceding claim, in which the substance retaining member (40) is a plate which is extended inside the reservoir (30) towards the closure element (50) by a preferably conical first rod (71) whose end (72) co-operates with the closure element (50).

7. A device according to claim 6, in which said air blaster (20) further comprises a second rod (75) which co-operates with said retaining member (40) to move it together with the first rod (71) when the device is actuated.

8. A device according to claim 7, in which said second rod (75) is provided with at least one external air passageway groove (73).

9. A device according to any preceding claim, in which the substance retaining member (40) is provided with one or more air passageway grooves.

10. A device according to any preceding claim, in which the reservoir (30) comprises a powder reservoir (35) and a liquid reservoir (36), and the substance retaining member (40) comprises a first retaining member (45) which, at rest, separates the two reservoirs (35, 36), and a second retaining member (46), the mechanical opening system (80) comprising a rod (81) interconnecting said two retaining members (45, 46) so that, when the rod (81) moves, the liquid is firstly transferred into the powder reservoir (35) to mix with the powder therein, the closure element (50) being ejected mechanically at the end of the stroke (81) of the rod by said first retaining member (45), air passageway means (83) being opened to connect the air blaster to the powder reservoir (35) so as to deliver the powder and liquid mixture through said substance outlet (32).

11. A device according to any preceding claim, in which said mechanical opening system (60, 70, 80) forms a guide system for manually actuating the air blaster (20).

12. A device according to any preceding claim, in which said air blaster (20) comprises a piston (21) mounted to slide in an air chamber (22).

13. A device according to any one of claims 1 to 11, in which said air blaster comprises a bellows (25).

## Patentansprüche

1. Ausgabevorrichtung für ein flüssiges oder pulverförmiges Produkt, aufweisend eine Ausgabeöffnung (10), einen Luftstoß (20) zum Erzeugen eines Luftstroms bei Betätigung der Vorrichtung, und mindestens einen Behälter (30), der eine einzelne Produktdosis enthält, wobei der Behälter (30) einen Lufteinlass (31), der mit dem Luftstoß (20) verbunden ist, und einen Produktauslass (32), der mit der Ausgabeöffnung (10) verbunden ist, aufweist, wobei der Lufteinlass (31) eine Produktrückhalteeinrichtung (40) aufweist, um das Produkt bis zur Ausgabe des Produktes in dem Behälter (30) zu halten, und wobei der Produktauslass (32) durch ein Verschlusselement (50) verschlossen ist, wobei die Vorrichtung ein mechanisches Öffnungssystem (60, 70, 80) aufweist, das mit dem Verschlusselement (50) zusammenwirkt, um es bei Betätigung der Vorrichtung mechanisch aus seiner Verschlussposition zu drängen, **dadurch gekennzeichnet, dass** das Verschlusselement (50) ein kugelförmiges Element, so wie eine kleine Kugel, ist, die mittels Kraft in den Produktauslass (32) des Behälters (30) gepresst wird, wobei das mechanische Öffnungssystem (70) mit der Produktrückhalteeinrichtung (40) einstückig ausgebildet ist.

2. Vorrichtung nach Anspruch 1, wobei das mechanische Öffnungssystem (60, 70, 80) mit dem Luftstoß (20) einstückig ausgebildet ist.

3. Vorrichtung nach Anspruch 1 oder 2, wobei die Produktrückhalteeinrichtung (40) vor Betätigung der Vorrichtung gegenüber dem Produkt und gegenüber Luft undurchlässig ist.

4. Vorrichtung nach Anspruch 1 oder 2, wobei die Produktrückhalteeinrichtung (40) vor Betätigung der Vorrichtung gegenüber dem Produkt undurchlässig und gegenüber Luft durchlässig ist.

5. Vorrichtung nach einem der vorhergehenden Ansprüche, wobei das mechanische Öffnungssystem (60) einen beweglichen Schaft (61, 62) aufweist, der bei Betätigung der Vorrichtung geeignet ist, den Behälter (30) zu durchqueren, um das Verschlusselement (50) zu verdrängen.

6. Vorrichtung nach einem der vorhergehenden Ansprüche, wobei die Produktrückhalteeinrichtung (40) eine Platte ist, die sich in dem Behälter (30) in Richtung zum Verschlusselement (50) durch einen ersten Schaft (71) fortsetzt, der vorzugsweise konisch ist, und dessen Ende (72) mit dem Verschlusselement (50) zusammenwirkt.

7. Vorrichtung nach Anspruch 6, wobei der Luftstoß (20) einen zweiten Schaft (75) aufweist, der mit der Rückhalteeinrichtung (40) zusammenwirkt, um sie bei Betätigung der Vorrichtung zusammen mit dem ersten Schaft (71) zu verschieben.

8. Vorrichtung nach Anspruch 7, wobei der zweite Schaft (75) mindestens eine äußere Nut (73) als Luftdurchlass aufweist.

9. Vorrichtung nach einem der vorhergehenden Ansprüche, wobei die Produktrückhalteeinrichtung (40) eine oder mehrere Nuten als Luftdurchlass aufweist.

10. Vorrichtung nach einem der vorhergehenden Ansprüche, wobei der Behälter (30) einen Pulverbehälter (35) und einen Flüssigkeitsbehälter (36) aufweist, und die Produktrückhalteeinrichtung (40) eine erste Rückhalteeinrichtung (45), die in Ruhestellung die beiden Behälter (35, 36) trennt, und eine zweite Rückhalteeinrichtung (46) aufweist, wobei das mechanische Öffnungssystem (80) einen Schaft (81) aufweist, der die beiden Rückhalteeinrichtungen (45, 46) derart miteinander verbindet, dass die Flüssigkeit bei Verschieben des Schaftes (81) zunächst in den Pulverbehälter (35) überführt wird, um sich dort mit dem Pulver zu vermischen, wobei das Verschlusselement (50) am Ende des Hubs des Schafts (81) durch die erste Rückhalteeinrichtung (45) mechanisch verdrängt wird, wobei Luftdurchlassmittel (83) geöffnet sind, um den Luftstoß mit dem Pulverbehälter (35) zu verbinden, um das Austreiben der Mischung aus Pulver und Flüssigkeit über den Produktauslass (32) zu verwirklichen.

11. Vorrichtung nach einem der vorhergehenden Ansprüche, wobei das mechanische Öffnungssystem (60, 70, 80) eine Führung zur manuellen Betätigung des Luftstoßes (20) bildet.

12. Vorrichtung nach einem der vorhergehenden Ansprüche, wobei der Luftstoß (20) einen Kolben (21) aufweist, der in einer Luftkammer (22) gleitet.

13. Vorrichtung nach einem der Ansprüche 1 bis 11, wobei der Luftstoß einen Blasebalg (25) aufweist.
